# EUROPÄISCHE PATENTSCHRIFT

(11) **EP 1 544 192 B1**
(45) Veröffentlichungstag und Bekanntmachung des Hinweises auf die Patenterteilung: **04.03.2009**
(21) Anmeldenummer: 04028658.5
(22) Anmeldetag: 03.12.2004
(51) Int. Cl.: C07C 211/25, C07C 17/16, C07C 22/08, C07B 39/00, B01J 31/02

(54) **Fluorierungsreagenzien enthaltend 1-Fluor-1-aminoolefine, deren Herstellung und Verwendung**
Reagents for fluorination containing 1-fluoro-1-aminoolefins, their synthesis and use
Réactifs de fluoration contenant des 1-fluoro-1-aminooléfines, leur synthèse et utilisation

(30) Priorität: 18.12.2003 DE 10359629
(43) Veröffentlichungstag der Anmeldung: 22.06.2005
(73) Patentinhaber: Saltigo GmbH, 40764 Langenfeld (DE)
(72) Erfinder: Ebenbeck, Wolfgang, Dr., 51373 Leverkusen (DE); Marhold, Albrecht, Dr., 51373 Leverkusen (DE)
(74) Vertreter: Wichmann, Birgid

(56) Entgegenhaltungen:
- EP-A- 0 373 118
- EP-A- 0 895 991
- L. GHOSEZ ET AL: "alpha-chloro enamines, reactive intermediates for synthesis: 1-chloro-N,N,2-trimethylpropenylamine" ORGANIC SYNTHESES, Bd. 59, 1979, Seiten 26-34, XP009046416
- L. GHOSEZ ET AL: "Synthesis of acyl halides under very mild conditions" J.CHEM.SOC.CHEM.COMM., 1979, Seiten 1180-1181, XP009046410
- FALBE J ED - HOUBEN-WEYL (HERAUSGEBER E MULLER): "Carbonsäuren und Carbonsäure-Derivate" CARBONSAEUREN UND CARBONSAEURE-DERIVATE, METHODEN DER ORGANISCHEN CHEMIE, STUTTGART, G. THIEME VERLAG, DE, Bd. TEIL 1 BAND E 5, 1985, Seiten 593-600, XP002226389
- "Organic Reactions" 1974, JOHN WILEY & SONS, INC , USA , XP002325124 ISBN: 0-471-19622-3 *Volume 21, p.158-159*

## Beschreibung

Die vorliegende Erfindung betrifft Fluorierungsreagenzien enthaltend 1-Fluor-1-aminoolefine sowie Verfahren zur Herstellung von Fluorierungsreagenzien enthaltend 1-Fluor-1-aminoolefine.

Als Reagenzien zur Fluorierung von Alkoholen oder Carbonylverbindungen wie insbesondere Ketonen, Carbonsäuren und Aldehyden sind beispielsweise Schwefeltetrafluorid, Diethylaminoschwefeltrifluorid (DAST) und Bis-(methoxyethyl)-aminoschwefeltrifluorid (Methoxy-DAST) bekannt (siehe auch US 3,976,691, EP-A 90 448 und EP-A 905 109). Nachteilig am industriellen Einsatz von Schwefeltetrafluorid ist dessen extrem hohe Toxizität und die Notwendigkeit von umfangreichen Sicherheitsmaßnahmen, die genannten Aminoschwefeltrifluoride sind darüber hinaus stoßempfindlich (J. Fluorine Chem. 1989, 42, 137) und unterliegen aufgrund ihrer Explosivität strengen gesetzlichen Auflagen.

Ein weiteres Reagenz zur Fluorierung von sekundären Alkoholen und Carbonsäuren ist N,N-Dimethyl-1,1-difluorbenzylamin, das durch Umsetzung von N,N-Dimethylbenzamid mit Schwefeltetrafluorid bei 150°C erhältlich ist (J. Fluorine Chem., 1983, 23, 219-228). Das Reagenz ist jedoch in seiner Anwendungsbreite beschränkt und liefert nur mittlere Ausbeuten.

Weiterhin ist als Fluorierungsmittel für Alkohole 2-Chlor-1,1,2-trifluortriethylamin, das sogenannte Yarovenko-Reagenz bekannt (Org. React. 1974, 21, 158). Das Reagenz ist jedoch nicht lagerbeständig und nur unter großem Aufwand herstellbar.

Ein ähnliches Reagenz, das unter dem Namen Ishikawa-Reagenz geläufig ist, besteht aus einer Mischung von Hexafluorpropyl-dialkylamin und Pentafluoralkenyl-dialkylamin, weist jedoch die gleichen oben genannten Nachteile auf.

Aus EP-A 895 991 sind Difluormethylen-α,α-diazoverbindungen bekannt, die zur Fluorierung von Hydroxy- und Carboxylfunktionen eingesetzt werden können. Aufgrund ihrer hohen Empfindlichkeit gegen Luft und Feuchtigkeit sind sie für den technischen Einsatz jedoch nur bedingt geeignet.

EP-A-0 373 118 (CIBA-GEIGY AG) offenbart die Herstellung geschützter Mono- und Oligozuckerhalogenide durch Halogenierung der geschützten Mono- und oligosaccharide. In den Beispielen 4, 8, 11, 13, 17 und 21 werden flurorierte saccharide hergestellt, wobei die Hydroxylgruppe des saccharids mit 1-Fluor-N,N-diisopropyl-2-methylpropenylamin umgesetzt wird.

Es bestand daher das Bedürfnis, Fluorierungsreagenzien bereitzustellen, die sowohl effizient aus einfach verfügbaren Edukten hergestellt werden können, lagerstabil sind und die Hydroxy- und Ketofunktionen in guten Ausbeuten fluorieren können.

Es wurden nun Mischungen gefunden, enthaltend
- Verbindungen der Formel (I), 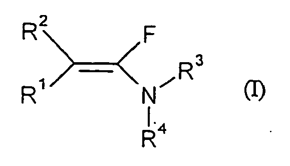 in der
   - R¹ und R²: jeweils unabhängig voneinander für C₁-C₁₂-Alkyl, C₁-C₁₂-Fluoralkyl, C₄-C₁₅-Aryl, C₅-C₁₆-Arylalkyl, oder [(C₂-C₁₂-Alkylen)-O]ₙ(C₁-C₁₂-alkyl)] mit n = 1 bis 5, stehen oder zusammen Teil eines cyclischen Restes mit insgesamt 3 bis 24 Kohlenstoffatomen sind und
   - R³ und R⁴: jeweils unabhängig voneinander für C₁-C₁₂-Alkyl, C₄-C₁₅-Arylalkyl oder C₅-C₁₆-Aryl stehen oder NR³R⁴ als Ganzes für einen cyclischen Aminorestes mit insgesamt 3 bis 18 Kohlenstoffatomen steht oder
   - R¹ und/oder R²: und R³ und/oder R⁴ gemeinsam Teil eines cyclischen Restes mit insgesamt 4 bis 18 Kohlenstoffatomen sind,
- zumindest ein, bevorzugt genau ein aprotisches, tertiäres Amin, das in α-Stellung zum Stickstoff keine Fluoratome enthält und/oder zumindest eine, bevorzugt genau eine N-heteroaromatische Verbindung und
- Fluorwasserstoff.

Aprotisch bedeutet in diesem Zusammenhang, dass das tertiäre Amin, das auch ein Molekül mit mehreren tertiären Aminogruppen sein kann, keine Wasserstoffatome trägt die bezogen auf eine wässrige Vergleichsskala bei 25°C einen pKs-Wert von weniger als 20 aufweisen.

Es sei angemerkt, dass unter den oben aus Vereinfachungsgründen gewählten Begrifflichkeiten auch die entsprechenden tertiären Ammoniumfluoride und N-Heteroaryliumfluoride und die entsprechenden Polyfluoride umfasst sind, wie sie bei der Reaktion mit Fluorwasserstoff auftreten.

Der Rahmen der Erfindung umfasst auch Kombinationen aller aufgeführten, allgemeinen oder in Vorzugsbereichen genannten Restedefinitionen und Parameter untereinander, also auch beliebige Kombinationen zwischen den jeweiligen Bereichen und Vorzugsbereichen.

Alkyl beziehungsweise Alkylen beziehungsweise Alkoxy steht jeweils unabhängig für einen geradkettigen, cyclischen, verzweigten oder unverzweigten Alkyl beziehungsweise Alkylen beziehungsweise Alkoxy-Rest. Gleiches gilt für den nichtaromatischen Teil eines Arylalkyl-Restes.

C₁-C₄-Alkyl steht beispielsweise für Methyl, Ethyl, n-Propyl, iso-Propyl, n-Butyl, sec.-Butyl und tert.-Butyl, C₁-C₈-Alkyl darüber hinaus beispielsweise für n-Pentyl, 1-Methylbutyl, 2-Methylbutyl, 3-Methylbutyl, neo-Pentyl, 1-Ethylpropyl, cyclo-Hexyl, cyclo-Pentyl, n-Hexyl, 1,1-Dimethylpropyl, 1,2-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, 1-Ethyl-2-methylpropyl, n-Heptyl und n-Octyl, C₁-C₁₂-Alkyl weiter darüber hinaus beispielsweise für Adamantyl, die isomeren Menthyle, n-Nonyl, n-Decyl und n-Dodecyl.

C₁-C₄-Alkoxy steht beispielsweise für Methoxy, Ethoxy, n-Propoxy, iso-Propoxy, n-Butoxy, sec.-Butoxy und tert.-Butoxy, C₁-C₈-Alkoxy darüberhinaus für n-Pentoxy, 1-Methylbutoxy, 2-Methylbutoxy, 3-Methylbutoxy, neo-Pentoxy, 1-Ethylpropoxy, cyclo-Hexoxy, cyclo-Pentoxy, n-Hexoxy und n-Octoxy, C₁-C₁₂-Alkoxy weiter darüber hinaus beispielsweise für Adamantoxy, die isomeren Menthoxy-Reste, n-Decoxy und n-Dodecoxy.

C₂-C₁₂-Alkylen steht beispielsweise für 1,2-Ethylen, 1,3-Propylen, 1,4-Butylen, 1,2-cyclo-Hexoxylen und 1,2-cyclo-Pentylen.

Aryl steht beispielsweise für carbocyclische aromatische Reste mit 6 bis 18 Gerüstkohlenstoffatomen oder heteroaromatische Reste mit 5 bis 18 Gerüstkohlenstoffatomen, in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können. Weiterhin können die carbocyclischen aromatischen Reste oder heteroaromatische Reste mit bis zu fünf gleichen oder verschiedenen Substituenten pro Cyclus substituiert sein, ausgewählt aus der Gruppe Chlor, Fluor, Cyano, C₁-C₁₂-Alkyl, C₆-C₁₂-Aryl, wie zum Beispiel Phenyl und C₁-C₆-Alkoxy. Beispiele für carbocyclische aromatische Reste mit 6 bis 18 Gerüstkohlenstoffatomen sind zum Beispiel Phenyl, Naphtyl, Phenanthrenyl, Anthracenyl oder Fluorenyl, heteroaromatische Reste mit 5 bis 18 Gerüstkohlenstoffatomen in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können sind beispielsweise Pyridinyl, Oxazolyl, Thiophen-yl, Benzofuranyl, Benzothiophen-yl, Dibenzofuran-yl, Dibenzothiophen-yl, Furanyl, Iudolyl, Pyridazinyl, Pyrazinyl, Pyrimidinyl, Thiazolyl, Triazolyl oder Chinolinyl.

Arylalkyl steht jeweils unabhängig für einen geradkettigen, cyclischen, verzweigten oder unverzweigten Alkyl-Rest nach vorstehender Definition, der einfach, mehrfach oder vollständig durch Aryl-Reste gemäß vorstehender Definition substituiert ist.

Im Folgenden werden die bevorzugten Substitutionsmuster für Verbindungen der Formel (I) definiert:
- R¹ und R²: stehen bevorzugt jeweils unabhängig für C₁-C₈-Alkyl, oder C₅-C₁₄-Aryl oder sind zusammen Teil eines monocyclischen C₅-C₁₂-Alkylrestes.
- R¹ und R²: stehen besonders bevorzugt jeweils unabhängig noch weiter bevorzugt jeweils identisch für Methyl, Phenyl oder sind zusammen Teil eines Furanyliden, Tetrahydronaphthalinyden oder Cyclohexylidenresten.
- R³ und R⁴: stehen bevorzugt jeweils unabhängig für C₁-C₁₂-Alkyl, oder C₅-C₁₄-Aryl oder NR³R⁴ ist als Ganzes ein monocyclischer C₄-C₁₂-Alkylrest.
- R³ und R⁴: stehen besonders bevorzugt jeweils unabhängig voneinander noch weiter bevorzugt jeweils identisch für Methyl, Ethyl, Isopropyl, oder NR³R⁴ als Ganzes für N-Morpholinyl, N-Methyl-1,4-Piperazin-N-yl und Prolinyl.

Als besonders bevorzugte Verbindungen der Formel (I) seien genannt:
2,2-Dimethyl-1-fluor-1-diisopropylaminoethylen, 2,2-Cyclohexyliden-1-fluor-1-piperidino-ethylen, 2,2-Diphenyl-1-fluordimethylamino-ethylen, 2,2-(3-Furanyliden)-1-fluor-1-Diethylamino-ethylen und 2,2-(2-Tetrahydro-naphthalinyliden)-1-fluor-1-prolino-ethylen.

Bevorzugte aprotische tertiäre Amine sind solche der Formeln (IIa) und (IIb),
NR⁵R⁶R⁷ (IIa)
(R⁸)₂N-L-N(R⁸)₂ (IIb)
in denen R⁵, R⁶ und R⁷ jeweils unabhängig voneinander für C₁-C₁₂-Alkyl oder [(C₂-C₁₂-Alkylen)-O]ₙ(C₁-C₁₂-alkyl)] mit n = 1 bis 5 stehen oder zwei oder drei der Reste R⁵, R⁶ und/oder R⁷ mit dem Stickstoffatom einen mono- bzw. bi-cyclischen Rest mit insgesamt 3 bis 12 bzw. 5 bis 15 Kohlenstoffatomen bilden, L für C₂-C₆-Alkylen steht und die Reste R⁸ jeweils unabhängig voneinander für C₁-C₈-Alkyl stehen oder zwei Reste zusammen für C₂-C₆-Alkylen stehen.

In Formel (IIa) stehen bevorzugt R⁵, R⁶ und R⁷ jeweils unabhängig voneinander für C₁-C₁₂-Alkyl, besonders bevorzugt jeweils identisch für C₁-C₈-Alkyl.

Besonders bevorzugte aprotische tertiäre Amine sind Triethylamin, Tetramethylethylendiamin und [2.2.2]-1,4-Diazabicyclooctan.

Bevorzugte N-heterocyclische Verbindungen sind gegebenenfalls substituierte Pyridine und Chinoline, wobei Pyridin besonders bevorzugt ist.

Im Rahmen der Erfindung ist der Einsatz von Triethylamin ganz besonders bevorzugt.

Das molare Verhältnis von aprotischem, tertiärem Amin oder N-heteroaromatischer Verbindung zu Verbindungen der Formel (I) beträgt beispielsweise und bevorzugt 0,1:1 bis 20:1, bevorzugt 1:1 bis 10:1 und besonders bevorzugt 1:1 bis 5:1.

Das molare Verhältnis von Fluorwasserstoff zu aprotischem, tertiärem Amin oder N-heteroaromatischer Verbindung beträgt beispielsweise und bevorzugt 0,2:1 bis 10:1 pro Stickstoffatom.

Die erfindungsgemäßen Mischungen enthaltend Verbindungen der Formel (I), zumindest ein aprotisches tertiäres Amin oder zumindest eine N-heteroaromatische Verbindung und Fluorwasserstoff sind beispielsweise durch Mischung der Verbindungen der Formel (I) mit aprotischem tertiären Amin oder N-heteroaromatischer Verbindung und Fluorwasserstoff erhältlich oder vorzugsweise durch Mischung der Verbindungen der Formel (I) mit Mischungen aus aprotischem, tertiärem Amin oder N-heteroaromatischer Verbindung und Fluorwasserstoff, die in verschiedenen Zusammensetzungen wie z.B. (NEt₃ x 3 HF) oder (Pyridin x 9HF) auch kommerziell erhältlich sind.

Die Verbindungen der Formel (I) lassen sich in besonders vorteilhafter Weise dadurch herstellen, dass Verbindungen der Formel (III), 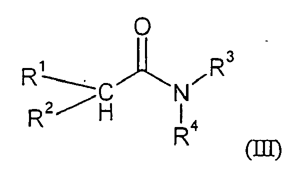 in der R¹, R², R³ und R⁴ die obenstehend angegebene Bedeutung einschließlich der genannten Vorzugsbereiche besitzen
- in einem Schritt a) in Verbindungen der Formeln (IVa) oder (IVb) überführt werden 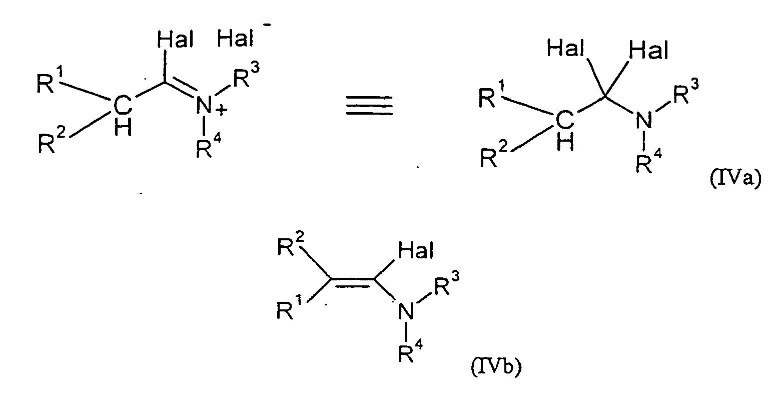 in der Hal jeweils unabhängig für Chlor, Brom oder Fluor steht und
- für den Fall, dass Hal für Chlor oder Brom steht in einem Schritt b) die Verbindungen der Formeln (IVa) und (IVb) mit ionischem Fluorid in Verbindungen der Formel (IVa) und (IVb) überführt werden in der Hal für Fluor steht und
- in einem Schritt c) die Verbindungen der Formel (IVa) und (IVb), in der Hal für Fluor steht, destilliert werden.
Anschließend können
- in einem Schritt d) die Verbindungen der Formel (I) mit zumindest einem aprotischen tertiären Amin oder zumindest einer N-heteroaromatischen Verbindung und Fluorwasserstoff oder Mischungen aus zumindest einem aprotischen tertiären Amin oder N-heteroaromatischen Verbindung und Fluorwasserstoff zu den erfindungsgemäßen Mischungen umgesetzt werden.

Bevorzugte Halogenierungsmittel für Schritt a) sind Phosphorpentachlorid, Phosphorpentabromid, Thionylchlorid, Thionylbromid, Phosgen und/oder Oxalsäurechlorid, wobei Phosphorpentachlorid, Thionylchlorid, Phosgen und/oder Oxalsäurechlorid noch weiter bevorzugt sind.

Das molare Verhältnis von Halogenierungsmittel zu Verbindung der Formel (III) beträgt beispielsweise und bevorzugt 0,9:1 bis 10:1, bevorzugt 1:1 bis 2:1 und besonders bevorzugt 1,02:1 bis 1,5:1.

Als Lösungsmittel für Schritt a) können aliphatische, alicyclische oder aromatische, gegebenenfalls halogenierte Kohlenwasserstoffe, wie beispielsweise Benzin, Benzol, Toluol, Xylol, Chlorbenzol, die isomeren Dichlorbenzole, Petrolether, Hexan, Cyclohexan, Dichlormethan, Chloroform und/oder Ether, wie Diethylether, Diisopropylether, Dioxan, Tetrahydrofuran oder Ethylenglykoldimethyl- oder -diethylether eingesetzt werden.

Die Reaktionstemperatur in Schritt a) kann beispielsweise -20°C bis zum Siedepunkt des eingesetzten Lösungsmittel bei Reaktionsdruck betragen maximal jedoch 150°C, bevorzugt -10°C bis zum Siedepunkt des eingesetzten Lösungsmittel bei Reaktionsdruck, maximal jedoch 50°C.

Der Reaktionsdruck in Schritt a) kann beispielsweise 0,8 bis 20 bar betragen, bevorzugt sind 0,9 bis 3 bar, wobei Umgebungsdruck noch weiter bevorzugt ist.

Die Aufarbeitung nach der Reaktion kann z. B. durch Abdestillieren aller flüchtigen Bestandteile und Trocknen des Rückstandes im Hochvakuum erfolgen.

Gemäß Schritt b) werden die Verbindungen der Formel (VI) mit ionischem Fluorid umgesetzt.

Ionische Fluoride sind beispielsweise quartäre Ammonium- oder Phosphoniumfluoride sowie Alkalimetallfluoride oder Mischungen der genannten Verbindungen.

Beispiele für Ammonium- oder Phosphoniumfluoride sind solche der Formel (V),
(Kation⁺)(F⁻) (V)
in der
(Kation⁺) für Kationen der Formel (VI) steht
[Pnyc(C₁-C₁₂-Alkyl)_{q}(C₆-C₁₅-Arylalkyl)ᵣ(C₃-C₁₄-Aryl)ₛ({(C₂-C₈-Alkylen)-O]ᵥ-(C₁-C₈-Alkyl)}ₜ)]⁺ (VI)
wobei
- Pnyc: für Stickstoff oder Phosphor steht und
in der (q+r+s+t) = 4 ist.

Bevorzugt ist jedoch der Einsatz von Alkalimetallfluoriden oder Mischungen von Alkalimetallfluoriden, wobei Natrium-, Kalium- und Cäsiumfluorid besonders bevorzugt und Natriumfluorid ganz besonders bevorzugt ist.

Das molare Verhältnis von ionischem Fluorid zu eingesetzter Verbindung der Formel (IVa) oder (IVb) kann beispielsweise 0,7 bis 5 betragen, vorzugsweise 0,9 bis 2 und besonders bevorzugt 1,1 bis 1,7. Die einsetzbare Menge an ionischem Fluorid ist nach oben lediglich durch wirtschaftliche Überlegungen begrenzt.

Vorzugsweise wird Schritt b) in einem organischen Lösungsmittel durchgeführt. Als organische Lösungsmittel sind beispielsweise geeignet: Nitrile, wie Acetonitril, Propionitril, Benzonitril, Benzylnitril oder Butyronitril; Amide wie N,N-Dimethylformamid, N,N-Dimethylacetamid, N-Methyl-formanilid, N-Methyl-pyrrolidon und Dimethylimidazolidinon sowie die als Ausgangsverbindungen für die Herstellung der Verbindungen der Formel (VI) eingesetzten Amide oder Sulfoxide, wie Dimethylsulfoxid, Sulfone wie Tetramethylensulfon, Polyether wie 1,4-Dioxan, Ethylenglykoldimethylether, Ethylenglykoldiethylether, Diethylenglykoldimethylether, Diethylenglykoldiethylether, Benzotrifluoride oder Gemische solcher organischen Lösungsmittel.

Vorzugsweise beträgt der Wassergehalt des Lösungsmittels im erfindungsgemäßen Verfahren maximal 0,2 Gew.-%, bevorzugt maximal 0,05 Gew.-%. Vorzugsweise wird ein solcher Wassergehalt durch Andestillieren oder Trocknung in an sich bekannter Weise erreicht. Bei Einsatz von Alkalimetallfluoriden wird besonders bevorzugt das Lösungsmittel gleichzeitig in Gegenwart des eingesetzten Alkalimetallfluorids getrocknet bzw. andestilliert.

Die Reaktionstemperatur in Schritt b) kann beispielsweise 60°C bis zum Siedepunkt des eingesetzten Lösungsmittel bei Reaktionsdruck betragen maximal jedoch 180°C, bevorzugt 110°C bis zum Siedepunkt des eingesetzten Lösungsmittel bei Reaktionsdruck maximal jedoch 150°C betragen.

Der Reaktionsdruck kann beispielsweise 0,8 bis 30 bar betragen, bevorzugt sind 1 bis 2 bar.

Gegebenenfalls kann die Reaktivität der ionischen Fluoride durch Zusätze modifiziert werden. Geeignete Zusätze sind beispielsweise Phasentransferkatalysatoren.

Als Phasentransferkatalysatoren sind beispielsweise Kronenether, wie 18-Krone-6, 12-Krone-4, Dibenzo-18-Krone-6 oder Dibenzo-12-Krone-4, Kryptanden wie Kryptand[2.2.2] oder Podanden wie Polyglykolether oder solche der Formel (VII) geeignet,
(Kation⁺)(Anion⁻) (VII)
in der
- (Kation⁺): vorstehend genannte Bedeutung und Vorzugsbereiche besitzt und
- (Anion⁻): für das Anion einer organischen oder anorganischen Säure steht.

Gemäß Schritt c) werden die Verbindungen der Formel (I) durch Destillation, vorzugsweise bei 0,001 bis 800 mbar in hoher Ausbeute und Reinheit erhalten.

Alternativ zu dem oben angeführten Herstellungsverfahren für die Verbindungen der Formel (I) beziehungsweise den erfindungsgemäßen Mischungen kann zur Herstellung auch ein Verfahren angewandt werden, das dadurch gekennzeichnet ist, dass Verbindungen der Formel (III) mit der oben angegebenen Bedeutung in Gegenwart von Oxalylfluorid und/oder Difluorphosgen und gegebenenfalls organischem Lösungsmittel umgesetzt werden.

Als organische Lösungsmittel eignen sich die oben angeführten hier in gleicher Weise.

Das molare Verhältnis von Oxalylfluorid und/oder Difluorphosgen zu Verbindungen der Formel (I) beträgt dann beispielsweise und bevorzugt 0,8:1 bis 20:1, bevorzugt 1:1 bis 2:1 und besonders bevorzugt 1,02:1 bis 1,1:1. Der Einsatz größerer Mengen ist möglich, bringt jedoch keine Verbesserung der Ausbeute mit sich.

Die Reaktionstemperatur kann beispielsweise -50°C bis 100°C betragen, bevorzugt -10°C bis 50°C.

Der Reaktionsdruck kann dann beispielsweise 0,8 bis 20 bar betragen, bevorzugt sind 1,5 bis 5 bar.

Die Aufarbeitung nach der Reaktion kann z. B. durch Abdestillieren aller flüchtigen Bestandteile und Trocknen des Rückstandes im Hochvakuum erfolgen.

Auf erfindungsgemäße Weise werden die Verbindungen der Formel (I) in hoher Ausbeute und Reinheit erhalten.

Zur Herstellung der erfindungsgemäßen Mischungen kann dann, wie vorstehend beschrieben, Schritt d) durchgeführt werden.

Bevorzugt werden die erfindungsgemäßen Mischungen in einem Eintopfverfahren erhalten, wenn die Umsetzung der Verbindungen der Formel (III) mit Oxalylfluorid und/oder Difluorphosgen durchgeführt wird und zumindest ein aprotisches tertiäres Amin oder zumindest eine N-heteroaromatische Verbindung und Fluorwasserstoff oder Mischungen aus zumindest einem aprotischen tertiären Amin oder zumindest einer N-heteroaromatischen Verbindung und Fluorwasserstoff zugesetzt werden.

Die Zugabe von aprotischem tertiären Amin oder N-heteroaromatischer Verbindung dient dabei teilweise dem Abfangen von Fluorwasserstoff, die Zugabe der Mischung aus zumindest einem aprotischen tertiären Amin oder N-heteroaromatischen Verbindung und Fluorwasserstoff dem Einstellen eines optimalen Verhältnisses zwischen Verbindungen der Formel (I), aprotischem tertiären Amin oder N-heteroaromatischer Verbindung und Fluorwasserstoff.

Die Verbindungen der Formel (I) und insbesondere die erfindungsgemäßen Mischungen eignen sich besonders zur Herstellung von Fluorverbindungen aus den entsprechenden Hydroxyverbindungen sowie zur Herstellung von geminalen-Difluorverbindungen aus den entsprechenden Carbonylverbindungen.

Daher ist von der Erfindung auch ein Verfahren zur Herstellung von fluorhaltigen Verbindungen umfasst, das dadurch gekennzeichnet ist, dass Verbindungen enthaltend Hydroxy- und/oder Carbonylgruppen mit Verbindungen der Formel (I) und/oder den erfindungsgemäßen Mischungen umgesetzt werden.

Bevorzugte Verbindungen enthaltend Hydroxy- und/oder Carbonylgruppen sind solche, die zumindest eine aliphatische Hydroxygruppe und/oder zumindest eine Ketogruppe und/oder zumindest eine Aldehydgruppe und/oder eine Carboxylgruppe enthalten.

Besonders bevorzugte Verbindungen enthaltend Hydroxy- und/oder Carbonylgruppen sind solche, die eine aliphatische Hydroxygruppe oder eine Ketogruppe oder eine Aldehydgruppe oder eine Carboxylgruppe enthalten.

Die erfindungsgemäß herstellbaren fluorhaltigen Verbindungen eignen sich insbesondere zur Herstellung von Arzneimitteln, Agrochemikalien und Flüssigkristallen.

Die Verbindungen der Formel (I) und die erfindungsgemäßen Mischungen besitzen den Vorteil, dass sie einfach herzustellen und lagerstabil sind und die Umsetzung von Hydroxy- und Carbonylverbindungen zu den entsprechenden Fluor- und/oder Difluorverbindungen in hohen Ausbeuten ermöglichen. Die erfindungsgemäßen Verfahren zur Herstellung der oben genannten Verbindungen und Mischungen gehen von leicht verfügbaren Edukten aus und liefern die Produkte in hohen Ausbeuten.

### Beispiele:

### Beispiel 1

### Herstellung eines Fluorierungsreagenzes enthaltend 2,2-Cyclohexyliden-1-fluor-1-diisopropylaminoethylen

In einem trockenen Autoklaven wurden 200 ml trockenes Dichlormethan vorgelegt und anschließend 63,3 g Cyclohexancarbonsäure-diisopropylamid zugefügt. Nach Verschließen des Autoklavens wurde auf 0°C gekühlt und dann unter Rühren 35 g Oxalylfluorid in kleinen Portionen in die Lösung eingedrückt. Es wurde für 8 Stunden bei 20°C nachgerührt, dann die Gase über einen Wäscher entspannt und dann eine Mischung aus 33 g Triethylamintrishydrofluorid und 10,1 g Triethylamin hinzugefügt. Nachdem eine Stunde nachgerührt wurde, wurde das Dichlormethan bei einer Innentemperatur von 20 bis 25°C und einem leichten Vakuum abdestilliert.

Das Reagenz kann ohne weitere Reinigung eingesetzt werden.

### Beispiel 2

### Umsetzung eines Alkohols mit einem Fluorierungsreagenz enthaltend 2,2-Cyclohexyliden-1-fluor-1-diisopropylaminoethylen

In einem trockenen Polyethylengefäß wurde unter Schutzgasatmossphäre zu einer Lösung aus 4.3 g (11,5 mmol) der Mischung aus Beispiel 1 mit der angenäherten Formel 2,2-Cyclohexyliden-1-fluor-1-düsopropylaminoethylen * Triethylamin * 3 HF in 10 ml trockenem Dichlormethan in etwa 5 Minuten eine Lösung aus 1.3 g (9,6 mmol) 1-Phenyl-1-propanol in 5 ml trockenem Dichlormethan getropft. Man ließ den Ansatz 4 Stunden bei 20°C rühren. Danach wurden 20 ml Eiswasser zugefügt und die wässrige Phase zweimal mit je 20 ml Dichlormethan extrahiert. Die vereinigten organischen Phasen wurden über Natriumsulfat getrocknet, abfiltriert und einkonzentriert. Als Rückstand verblieben 1.11 g ( 8,0 mmol, 84 % d.Th.) 1-Fluor-1-phenylpropan als farblose Flüssigkeit.

## Patentansprüche

1. Mischungen enthaltend
• Verbindungen der Formel (I), 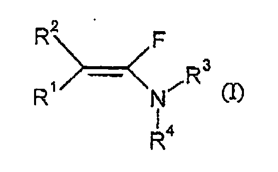 in der
R¹ und R² jeweils unabhängig voneinander für C₁-C₁₂-Alkyl, C₁-C₁₂-Fluoralkyl C₄-C₁₅-Aryl, C₅-C₁₆-Arylalkyl, oder [(C₁-C₁₂-Alkylen)-O]ₙ(C₁-C₁₂-alkyl)] mit n = 1 bis 5, stehen oder zusammen Teil eines cyclischen Restes mit insgesamt 3 bis 24 Kohlenstoffatomen sind und
R³ und R⁴ jeweils unabhängig voneinander für C₁-C₁₂-Alkyl, C₄-C₁₅-Arylalkyl oder C₅-C₁₆-Aryl stehen oder NR³R⁴ als Ganzes für einen cyclischen Aminorestes mit insgesamt 3 bis 18 Kohlenstoffatomen steht oder
R¹ und/oder R² und R³ und/oder R⁴ gemeinsam Teil eines cyclischen Restes mit insgesamt 4 bis 18 Kohlenstoffatomen sind,
• zumindest ein aprotisches, tertiäres Amin, das in α-Stellung zum Stickstoff keine Fluoratome enthält und/oder zumindest eine N-beteroaromatische Verbindung und
• Fluorwasserstoff.

2. Mischungen nach Anspruch 1 enthaltend genau ein aprotisches, tertiäres Amin, das in α-Stillung zum Stickstoff keine Fluoratome enthält.

3. Mischungen nach einem oder mehreren der Ansprüche 1 und 2, **dadurch gekennzeichnet, dass** in Formel (I)
R¹ und R² jeweils unabhängig für C₁-C₈-Alkyl, oder C₅-C₁₄-Aryl sterben oder zusammen Teil eines monocyclischen C₅-C₁₂-Alkylrestes sind.

4. Mischungen nach einem oder mehreren der Ansprüche 1 bis 3, **dadurch gekennzeichnet, dass** in Formel (I)
R¹ und R² jeweils unabhängig für Methyl oder Phenyl stehen oder zusammen Teil eines Furanyliden-, Tetrahydronaphthalinyden- oder Cyclohexylidenrestes sind.

5. Mischungen nach einem oder mehreren der Ansprüche 1 bis 4, **dadurch gekennzeichnet, dass** in Formel (I)
R¹ und R² jeweils identisch für Methyl oder Phenyl stehen oder zusammen Teil eines Furanyliden, Tetrahydronaphthalinyden oder Cyclohexylidenrestes sind.

6. Mischungen nach einem oder mehreren der Ansprüche 1 bis 5, **dadurch gekennzeichnet, dass** in Formel (I)
R³ und R⁴ jeweils unabhängig für C₁-C₁₂-Alkyl, oder C₅-C₁₄-Aryl stehen oder NR³R⁴ als Ganzes ein monocyclischer C₄-C₁₂-Alkylrest ist.

7. Mischungen nach einem oder mehreren der Ansprüche 1 bis 6, **dadurch gekennzeichnet, dass** in Formel (I)
R³ und R⁴ jeweils unabhängig voneinander für Methyl, Ethyl, oder Isopropyl stehen oder NR³R⁴ als Ganzes für N-Morpholinyl, N-Methyl-1,4-Piperazin-N-yl und Prolinyl steht.

8. Mischungen nach einem oder mehreren der Ansprüche 1 bis 7, **dadurch gekennzeichnet, dass** Formel (I) für
2,2-Dimethyl-1-fluor-1-diisopropylaminoethylen, 2,2-Cyclohexyliden-1-fluor-1-piperidino-ethylen, 2,2-Diphenyl-1-fluordimethylamino-ethylen, 2,2-(3-Furanyliden)-1-fluor-1-Diethylamino-ethylen und 2,2-(2-Tetrahydro-naphthalinyliden)-1-fluor-1-prolino-ethylen steht.

9. Mischungen nach einem oder mehreren der Ansprüche 1 bis 8, **dadurch gekennzeichnet, dass** aprotische tertiäre Amine solche der Formeln (IIa) und (IIb) sind
NR⁵R⁶R⁷ (IIa)
(R⁸)₂N-L-N(R⁸)₂ (IIb)
in denen R⁵, R⁶ und R⁷ jeweils unabhängig voneinander für C₇-C₁₂-Alkyl oder [(C₂-C₁₂-Alkylen)-O]ₙ(C₁-C₁₂-alkyl)] mit n = 1 bis 5 stehen oder zwei oder drei der Reste R⁵, R⁶ und/oder R⁷ mit dem Stickstoffatom einen mono- bzw. bicyclischen Rest mit insgesamt 3 bis 12 bzw. 5 bis 15 Kohlenstoffatomen bilden, L für C₂-C₆-Alkylen steht und die Reste R⁸ jeweils unabhängig voneinander für C₁-C₈-Alkyl stehen oder zwei Reste zusammen für C₂-C₆-Alkylen stehen.

10. Mischungen nach einem oder mehreren der Ansprüche 1 bis 9, **dadurch gekennzeichnet, dass** aprotische tertiäre Amine Triethylamin, Tetramethylethylendiamin und [2.2.2]-1.4-Diazabicyclooctan sind.

11. Mischungen nach einem oder mehreren der Ansprüche 1 bis 10, **dadurch gekennzeichnet, dass** das molare Verhältnis von aprotischem, tertiärem Amin oder N-heteroaromatischer Verbindung zu Verbindungen der Formel (I) 0,1: 1 bis 20:1 beträgt

12. Mischungen nach einem oder mehreren der Ansprüche 1 bis 11, **dadurch gekennzeichnet dass** das molare Verhältnis von Fluorwasserstoff zu aprotischem, tertiärem Amin oder N-heteroaromatischer Verbindung 0,2:1 bis 10:1 pro Stickstoffatom beträgt.

13. Verfahren zur Herstellung von Mischungen gemäß Anspruch 1. **dadurch gekennzeichnet, dass** Verbindungen der Formel (III) 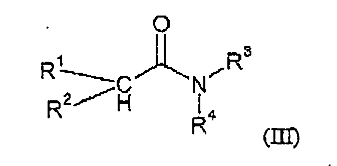 in der R¹, R², R³ und R⁴ die in Anspruch 1 angegebene Bedeutung einschließlich der genannten Vorzugsbereich besitzen, in Gegenwart von Oxalylfluorid und/oder Difluorphosgen und gegebenenfalls organischem Lösungsmittel umgesetzt werden und zumindest ein aprotisches tertiären Amin oder eine N-heteroaromatische Verbindung und Fluorowasserstoff oder Mischungen aus zumindest einem aprotischen tertiären Amin oder N-heteroaromatischen Verbindung und Fluorwasserstoff zugesetzt werden.

14. Verfahren zur Herstellung von fluorhaltigen Verbindungen, **dadurch gekennzeichnet, dass** Verbindungen enthaltend Hydroxy- und/oder Carbonylgruppen mit Mischungen gemäß einem oder mehreren der Ansprüche 1 bis 12 umgesetzt werden.

## Claims

1. Mixtures comprising
• compounds of the formula (I), 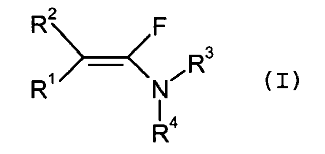
where
R¹ and R² are each, independently of one another, C₁-C₁₂-alkyl, C₁-C₁₂-fluoroalkyl, C₄-C₁₅-aryl, C₅-C₁₆-arylalkyl, or [(C₂-C₁₂-alkylene)-O]ₙ(C₁-C₁₂-alkyl)] where n = 1 to 5 or are together part of a cyclic radical having a total of from 3 to 24 carbon atoms and
R³ and R⁴ are each, independently of one another, C₁-C₁₂-alkyl, C₄-C₁₅-arylalkyl or C₅-C₁₆-aryl or the moiety NR³R⁴ is a cyclic amino radical having a total of from 3 to 18 carbon atoms or
R¹ and/or R² and R³ and/or R⁴ are together part of a cyclic radical having a total of from 4 to 18 carbon atoms,
• at least one aprotic, tertiary amine which has no fluorine atoms in the α-position relative to the nitrogen and/or at least one N-heteroaromatic compound and
• hydrogen fluoride.

2. Mixtures according to Claim 1 containing precisely one aprotic, tertiary amine which has no fluorine atoms in the α position relative to the nitrogen.

3. Mixtures according to one or more of Claims 1 and 2, **characterized in that**, in the formula (I),
R¹ and R² are each, independently of one another, C₁-C₈-alkyl or C₅-C₁₄-aryl or are together part of a monocyclic C₅-C₁₂-alkyl radical.

4. Mixtures according to one or more of Claims 1 to 3, **characterized in that**, in the formula (I),
R¹ and R² are each, independently of one another, methyl or phenyl or are together part of a furanylidene, tetrahydronaphthalenylidene or cyclohexylidene radical.

5. Mixtures according to one or more of Claims 1 to 4, **characterized in that**, in the formula (I),
R¹ and R² are identical and are each methyl or phenyl or are together part of a furanylidene, tetrahydronaphthalenylidene or cyclohexylidene radical.

6. Mixtures according to one or more of Claims 1 to 5, **characterized in that**, in the formula (I),
R³ and R⁴ are each, independently of one another, C₁-C₁₂-alkyl or C₅-C₁₄-aryl or the moiety NR³R⁴ is a monocyclic C₄-C₁₂-alkyl radical.

7. Mixtures according to one or more of Claims 1 to 6, **characterized in that**, in the formula (I),
R³ and R⁴ are each, independently of one another, methyl, ethyl or isopropyl or the moiety NR³R⁴ is N-morpholinyl, N-methyl-1,4-piperazin-N-yl or prolinyl.

8. Mixtures according to one or more of Claims 1 to 7, **characterized in that** the formula (I) represents
2,2-dimethyl-1-fluoro-1-diisopropylaminoethylene, 2,2-cyclohexylidene-1-fluoro-1-piperidinoethylene, 2,2-diphenyl-1-fluorodimethylaminoethylene, 2,2-(3-furanylidene)-1-fluoro-1-diethylaminoethylene or 2,2-(2-tetrahydronaphthalenylidene)-1-fluoro-1-prolinoethylene.

9. Mixtures according to one or more of Claims 1 to 8, **characterized in that** aprotic tertiary amines are compounds of the formulae (IIa) and (IIb)
NR⁵R⁶R⁷ (IIa)
(R⁸)₂N-L-N(R⁸)₂ (IIb)
where R⁵, R⁶ and R⁷ are each, independently of one another, C₁-C₁₂-alkyl or [(C₂-C₁₂-alkylene)-O]ₙ (C₁-C₁₂-alkyl)] where n = 1 to 5 or two or three of the radicals R⁵, R⁶ and/or R⁷ together with the nitrogen atom form a monocyclic or bicyclic radical having a total of from 3 to 12 or from 5 to 15 carbon atoms, L is C₂-C₆-alkylene and the radicals R⁸ are each, independently of one another, C₁-C₈-alkyl or two radicals together form C₂-C₆-alkylene.

10. Mixtures according to one or more of Claims 1 to 9, **characterized in that** aprotic tertiary amines are triethylamine, tetramethylethylenediamine and [2.2.2]-1,4-diazabicyclooctane.

11. Mixtures according to one or more of Claims 1 to 10, **characterized in that** the molar ratio of aprotic, tertiary amine or N-heteroaromatic compound to compounds of the formula (I) is from 0.1:1 to 20:1.

12. Mixtures according to one or more of Claims 1 to 11, **characterized in that** the molar ratio of hydrogen fluoride to aprotic, tertiary amine or N-heteroaromatic compound is from 0.2:1 to 10:1 per nitrogen atom.

13. Process for preparing mixtures according to Claim 1, **characterized in that** compounds of the formula (III) 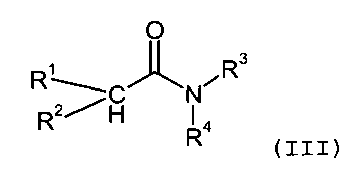 where R¹, R², R³ and R⁴ are as defined in Claim 1 including the preference ranges mentioned, are reacted in the presence of oxalyl fluoride and/or difluorophosgene and, if desired, an organic solvent with addition of at least one aprotic tertiary amine or an N-heteroaromatic compound and hydrogen fluoride or mixtures of at least one aprotic tertiary amine or N-heteroaromatic compound and hydrogen fluoride.

14. Process for preparing fluorine-containing compounds, **characterized in that** compounds containing hydroxyl and/or carbonyl groups are reacted with mixtures according to one or more of Claims 1 to 12.

## Revendications

1. Mélanges contenant
- des composés de formule (I) 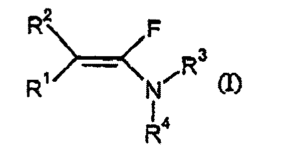
dans laquelle
R¹ et R² représentent, à chaque fois indépendamment l'un de l'autre, C₁-C₁₂-alkyle, C₁-C₁₂-fluoroalkyle, C₄-C₁₅-aryle, C₅-C₁₆-arylalkyle, ou [(C₂-C₁₂-alkylène)-O)]ₙ(C₁-C₁₂-alkyle)] avec n = 1 à 5, ou sont, ensemble, une partie d'un radical cyclique comprenant au total 3 à 24 atomes de carbone et
R³ et R⁴ représentent, à chaque fois indépendamment l'un de l'autre, C₁-C₁₂-alkyle, C₄-C₁₅-arylalkyle ou C₅-C₁₆-aryle ou NR³R⁴ est, dans sa totalité, un radical amino cyclique comprenant au total 3 à 18 atomes de carbone ou
R¹ et/ou R² et R³ et/ou R⁴ sont, ensemble, une partie d'un radical cyclique comprenant au total 4 à 18 atomes de carbone,
- au moins une amine tertiaire aprotique qui ne contient pas d'atomes de fluor en position α par rapport à l'azote et/ou au moins un composé N-hétéroaromatique et
- du fluorure d'hydrogène.

2. Mélanges selon la revendication 1, contenant exactement une amine tertiaire aprotique qui ne contient pas d'atomes de fluor en position α par rapport à l'azote.

3. Mélanges selon l'une ou plusieurs des revendications 1 et 2, **caractérisés en ce que**, dans la formule (I)
R¹ et R² représentent, à chaque fois indépendamment l'un de l'autre, C₁-C₈-alkyle ou C₅-C₁₄-aryle ou sont, ensemble, une partie d'un radical C₅-C₁₂-alkyle monocyclique.

4. Mélanges selon l'une ou plusieurs des revendications 1 à 3, **caractérisés en ce que**, dans la formule (I)
R¹ et R² représentent, à chaque fois indépendamment l'un de l'autre, méthyle ou phényle ou sont, ensemble, une partie d'un radical furannylidène, tétrahydronaphtalénylidène ou cyclohexylidène.

5. Mélanges selon l'une ou plusieurs des revendications 1 à 4, **caractérisés en ce que**, dans la formule (I)
R¹ et R² représentent, à chaque fois identiquement, méthyle ou phényle ou sont, ensemble, une partie d'un radical furannylidène, tétrahydronaphtalénylidène ou cyclohexylidène.

6. Mélanges selon l'une ou plusieurs des revendications 1 à 5, **caractérisés en ce que**, dans la formule (I)
R³ et R⁴ représentent, à chaque fois indépendamment l'un de l'autre, C₁-C₁₂-alkyle ou C₅-C₁₄-aryle ou NR³R⁴ est, dans sa totalité, un radical C₄-C₁₂-alkyle monocyclique.

7. Mélanges selon l'une ou plusieurs des revendications 1 à 6, **caractérisés en ce que**, dans la formule (I)
R³ et R⁴ représentent, à chaque fois indépendamment l'un de l'autre, méthyle, éthyle ou isopropyle ou NR³R⁴ est, dans sa totalité, N-morpholinyle, N-méthyl-1,4-pipérazin-N-yle et prolinyle.

8. Mélanges selon l'une ou plusieurs des revendications 1 à 7, **caractérisés en ce que** la formule (I) représente le 2,2-diméthyl-1-fluoro-1-diisopropylaminoéthylène, le 2,2-cyclohexylidène-1-fluoro-1-pipéridinoéthylène, le 2,2-diphényl-1-fluorodiméthylaminoéthylène, le 2,2-(3-furannylidène)-1-fluoro-1-diéthylaminoéthylène et le 2,2-(2-tétrahydronaphtalénylidène)-1-fluoro-1-prolinoéthylène.

9. Mélanges selon l'une ou plusieurs des revendications 1 à 8, **caractérisés en ce que** les amines tertiaires aprotiques sont des amines des formules (IIa) et (IIb)
NR⁵R⁶R⁷ (IIa)
(R⁸)₂N-L-N(R⁸)₂ (IIb)
dans lesquelles R⁵, R⁶ et R⁷ représentent, à chaque fois indépendamment l'un de l'autre, C₁-C₁₂-alkyle ou [(C₂-C₁₂-alkylène)-O]ₙ(C₁-C₁₂-alkyle)] avec n = 1 à 5 ou deux ou trois des radicaux R⁵, R⁶ et/ou R⁷, avec l'atome d'azote, forment un radical monocyclique ou bicyclique comprenant au total respectivement 3 à 12 ou 5 à 15 atomes de carbone, L représente C₂-C₆-alkylène et les radicaux R⁸ représentent, à chaque fois indépendamment l'un de l'autre, C₁-C₈-alkyle ou deux radicaux ensemble représentent C₂-C₆-alkylène.

10. Mélanges selon l'une ou plusieurs des revendications 1 à 9, **caractérisés en ce que** les amines tertiaires aprotiques sont la triéthylamine, la tétraméthyléthylènediamine et le [2.2.2]-1,4-diazabicyclooctane.

11. Mélanges selon l'une ou plusieurs des revendications 1 à 10, **caractérisés en ce que** le rapport molaire de l'amine tertiaire aprotique ou du composé N-hétéroaromatique aux composés de formule (I) est de 0,1:1 à 20:1.

12. Mélanges selon l'une ou plusieurs des revendications 1 à 11, **caractérisés en ce que** le rapport molaire du fluorure d'hydrogène à l'amine tertiaire aprotique ou au composé N-hétéroaromatique est de 0,2:1 à 10:1 par atome d'azote.

13. Procédé pour la préparation de mélanges selon la revendication 1, **caractérisé en ce qu'**on transforme des composés de formule (III) 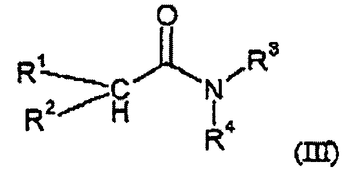 dans laquelle R¹, R², R³ et R⁴ présentent la signification indiquée dans la revendication 1, y compris les plages préférentielles mentionnées, en présence de fluorure d'oxalyle et/ou de difluorophosgène et le cas échéant d'un solvant organique et on ajoute au moins un amine tertiaire aprotique ou un composé N-hétéroaromatique et du fluorure d'hydrogène ou des mélanges d'au moins une amine tertiaire aprotique ou d'au moins un composé N-hétéroaromatique et de fluorure d'hydrogène.

14. Procédé pour la préparation de composés fluorés, **caractérisé en ce qu'**on transforme des composés contenant des groupes hydroxy et/ou carbonyle avec des mélanges selon l'une ou plusieurs des revendications 1 à 12.
